# EUROPEAN PATENT SPECIFICATION

(11) **EP 1 611 400 B1**
(45) Date of publication and mention of the grant of the patent: **13.09.2017**
(21) Application number: 04758633.4
(22) Date of filing: 31.03.2004
(51) Int. Cl.: A61F 7/03

(54) **SELF-CONTAINED PERSONAL WARMING APPARATUS**
UNABHÄNGIGE PERSONENWÄRMVORRICHTUNG
APPAREIL DE CHAUFFAGE AUTONOME POUR DES PERSONNES

(30) Priority: 01.04.2003 US 405668
(43) Date of publication of application: 04.01.2006
(73) Proprietor: Heatmax, Inc., Dalton, GA 30722 (US)
(72) Inventor: YIM, Daniel H., Dalton, GA 30721 (US)
(74) Representative: REHBERG HÜPPE + PARTNER
(86) International application number: PCT/US2004/009812
(87) International publication number: WO 2004/090435

(56) References cited:
- JP-A- 57 031 980
- JP-A- S57 031 980
- JP-A- S58 092 752
- JP-B- 7 090 030
- JP-U- 5 030 432
- US-A- 5 046 479
- US-A- 5 084 986
- US-A- 5 187 814
- US-A- 5 205 278
- US-A- 5 425 975
- US-A- 5 918 590
- US-A- 5 928 275

## Description

### TECHNICAL FIELD

The present invention is generally related to warming devices and, more particularly, is related to a self-contained personal warming apparatus and method of warming

### BACKGROUND OF THE INVENTION

Heat generating pouches of various configurations and shapes are designed and used for various purposes, such as hand warming, feet warming, and the like, by placing the heat generating pouch in a glove, mitten, shoe, *etc.* Heat generating pouches typically comprise a heat generating compound disposed between at least two layers of material, such as fabric, or the like, assembled to form a pouch. The heat generating compound emits heat during an exothermic chemical reaction resulting from exposure of the compound to air. Known heat generating compounds typically comprises a loose granular substance that is freely movable within the pouch. With a freely movable compound, when the pouch is placed flat, or horizontally, the compound is somewhat evenly distributed throughout the pouch. However, when the pouch is placed vertically, moved around, or jostled, the compound is drawn by gravity, shifts and settles toward one end of the pouch. This shifting and settling of the compound is sometimes referred to as a "tea-bag" effect. The tea-bag effect results in an uneven temperature profile along the surface area of the pouch. An uneven temperature profile can result in some areas not receiving heat, as desired, or an over concentration of heat in other areas.

The problem of the compound tending to shift and settle within the pouch has been addressed by other configurations of heat generating pouches. In one embodiment, the heat generating compound is contained within pucks or pellets that are disposed between at least two layers of material. The pucks or pellets comprise a heat generating compound capable of reacting with air in an exothermic reaction. The compound is compressed into concentrated, substantially rigid, pellets. In this configuration, however, the heat emission is concentrated at the pucks, resulting in an uneven heat distribution across the surface area of the pouch. Furthermore, because the pucks are rigid, the pucks do not conform to various contours of the human body against which the heat generating pouch may be placed.

The undesirable effect of a shifting compound has also been addressed by introducing air to the heat generating compound through only one of the two layers of material forming the pouch, while the other of the two layers of material comprises a self-adhesive. However, these adhesive pouches cannot be easily inserted into pockets formed in socks, gloves, mittens, specially designed belts, or the like for use. Indeed, such adhesive pouches are typically fixed to an interior surface of a user's clothing. In this configuration of use, the pouch moves away from the user's skin as the clothing moves away from the user's skin. Furthermore, fixing the pouch to a user's clothing typically results in minimal or no pressure being applied to the pouch as the pouch is applied to the user's skin, thereby rendering the pouch less effective.

Thus, a heretofore unaddressed need exists in the industry to address the aforementioned deficiencies and inadequacies.

US 5,046479 A discloses a disposable body warmer having the shape of a flat rectangular bag with a thickness of 2 to 5 mm. The bag has a rectangular shape with an area of 10 x 5 cm or more and 20 x 15 cm or less. The heating agent might comprise 55 to 65 % by weight of iron powder, 18 to 22 % by weight of water, 9 to 11 % by weight of water-retaining agent, 3.5 to 4.5 % by weight of activated carbon and about 4.5 to 6 % by weight of salt. The bag is provided with an air-permeable surface having an air permeability per unit of 5,000 to 10,000 sec./100 cc so as to bring about a reduction in the pressure accompanying oxidative heat generation of the heat generating agent packed in the bag. The amount of supply of oxygen is limited to a value less than that necessary for oxidation of the agent so that the above-described bag can be maintained in a compression-flattened state under atmospheric pressure during the oxidative heat generation. The temperature of heat generation is adjusted in such a manner that the maximum temperature is 65°C, preferably 62° to 63°C and the average temperature is 50° to 55°C. For one embodiment the bag produces an average temperature of 38° to 41°C for a duration of 20 to 24 hr. One surface of the bag is provided with a non-transferable self-adhesive layer so that the body warmer can be adhered to the underwear worn on the human body. The body warmer should keep its position also in a vertical state due to the self-adhesive layer. The self-adhesive effect is dimensioned such that the body warmer can be properly attached to any side of the human body without use of any additional attaching means. The body warmer is arranged in a wrapped, hermetically sealed outer bag which can be opened. The bag is made of flexible thermoplastic sheets and films of polyurethane, polypropylene and polyethylene and plastic and rubber materials prepared by a modification thereof or a non-woven fabric. An air permeability of the air-permeable surface of the bag is defined by interconnected micropores of a sheet or a film or by lamination of a resin film having interconnected micropores on a known non-woven fabric. The surface on which the non-transferable self-adhesive layer is provided may be essentially impermeable to air.

According to JP 07 090030 B the warming apparatus comprises a bag made of an air permeable film having a thickness of 10 µm or less and an airflow rate between 10,000 and 100,000 sec./100 cc. In use, an internal air pressure of the air permeable bag becomes a negative pressure. The heat generating agent of a prior art warming apparatus comprises iron powder. The bag is contained in an air impermeable outer bag which is broken for activating the iron powder in the inner bag. The heat generating agent comprises a catalyser. The bag is intended for being applied to the skin of a user without any undesired slip between the bag and the skin. JP 7 090030 B describes the problem to adjust the air flow rate of the air permeable surface for defining the desired temperature of the heat generating bag. The layers of the heat generating bag may include two laminated sublayers, namely an air-permeable film having superfine pores as an air permeable material and a rayon non-woven film adhered to the air permeable film. The oxygen in the air has to penetrate through the air permeable film as well as through the rayon non-woven fabric. The rayon non-woven fabric is used for assuring an agreeable feeling and an appropriate heat insulation property. The duration time of the heat generating process should be prolonged to 8 hours or more or 10 to 12 hours. For one embodiment disclosed one side of the bag is composed of the air permeable film and the rayon non-woven fabric, while the other side thereof is composed of an air impermeable film and the rayon non-woven fabric. The rationale behind this design is that heat is to be generated fast on the upper side of the bag while generation of heat of the lower side thereof is brought about slowly.

JP 05 30432 U suggests to create an air pressure inside a bag in the range of 5 to 60 mmHg. Here, a part or the whole of the air permeable surface is a porous film or a composite material composed of a porous film and an air permeable wrapping material. The bag might comprise an additional air venting material of which a pore diameter is larger than that of an air venting material of the porous film. For adjusting the air permeability a part of the porous film or a part of the composite material composed of the porous film and the air permeable wrapping material are thermally fused. The porous film might be polyethylene, polypropylene, ethylene/vinyl acetate copolymer. The air permeable inner bag is contained in an outer bag made of an air impermeable film. JP 5 30432 U discloses to define the rate of supply of oxygen to the heat generating agent by varying the area, shape, number and arrangement of air venting materials. JP 5 30432 U further describes prior art wherein the air permeability is adjusted by applying an air impermeable coating agent such as an adhesive to an air permeable wrapping material. JP 5 30432 U discloses an embodiment wherein a part or the whole of a porous film wrapping material comprises a porous film or which is a composite material of a porous film and an air permeable wrapping material. The permeability is adjusted by applying a thermal fusion partially to the desired portion with thermally fused portions and a non-thermally fused portion. The air impermeable surface comprises an air permeable wrapping material prepared by bonding an air impermeable wrapping film and a non-woven fabric one upon another. The porous wrapping material might be made of a thermoplastic sheet or film having continuous micropores and being made of plastics such as polyethylene, polypropylene, ethylene-vinyl acetate copolymer, polyurethane or improved soft plastics or rubbers of these plastics. The heat generating agent is composed of a metallic powder such as iron powder, water, an oxidation assistant such as sodium chloride, and a water-retaining agent such as wood powder, vermiculite, activated carbon, a composition mainly composed of alkali metal sulfide, polysulfide or hydrate salts thereof and a carbonaceous material and/or iron carbide. For one embodiment a porous film with an air permeability of 4,000 sec./100 ml is bonded to a polyethylene made porous film having a permeability of 850 sec./100 ml. The heat generating agent might be prepared by mixing 60 parts of iron powder, 25 parts of 10 % salt water, 13 parts of activated carbon, 14 parts of wood powder. The bag produces a maximum temperature of 61°C to 63°C with a duration of 7 to 8 hours. For an alternative embodiment the porous film wrapping material was a laminated film having a permeability of 5,000 sec./100 ml bonded to a polyethylene made porous film having a maximum pore diameter of 0,7 µm and a permeability of 2,800 sec./100 ml and a nylon-non-woven fabric.

US 5,918,590 A discloses prior art wherein a thermal pack creates a temperature between 38°C to 41°C for treating temporary or chronic pain. US 5,918,590 A discloses to use a heat generating agent in a heat cell comprising about 30 % to 80 % iron powder, 3 % to 25 % activated carbon, non-activated carbon and mixtures thereof, 0.5 % to 10 % metal salt and 1 % to 40 % water. A bag comprises film layer substrates made of films or films laminated to non-woven fabrics. Examples for suitable films include polyethylene, polypropylene, nylon, polyester, polyvinyl chloride, polyvinylidene chloride, polyurethane, polystyrene, saponified ethylene-vinyl acetate copolymer, ethylene-vinyl acetate copolymer, natural rubber, reclaimed rubber and synthetic rubber. The opposed substrates are created by bonding two substrates together around their periphery thereby forming a pouch, envelope or pocket wherein the non-woven side faces to the outside. The resulting heat cell might have the shape of a disk, a triangle, a sphere, a square, e cube, a rectangular or cylindrical shape with a total surface area below about 50 cm². The desired permeability properties are provided by microporous films or by films which have pores or holes or perforations formed therein. The velocity, duration and temperature of the thermogenic oxidation reaction of the composition can be controlled as desired by changing the area of contact with air, more specifically, by changing the oxygen diffusion/permeability. The finished heat cell is packed in an outer secondary air impermeable package to prevent the oxidation reaction from occurring until desired. Alternatively, air impermeable removable adhesive strips can be placed over the aeration holes in the heat cells such that, when the strips are removed, air is allowed to enter the heat cell, thus activating the oxidation reaction of the iron powder. US 5,918,590 A discloses examples wherein the percentage of iron in the heat generating agent is 55.2 % or 74 %.

US 5,425,975 discloses a heat-generating body comprising a sheet-shaped substrate comprising irregularly arranged fibers and having a multiplicity of gaps among the fibers. An oxidizable metal powder is dispersed in and supported by the substrate. An inorganic electrolyte solution contains activated carbon mixed and suspended therein. The substrate is impregnated with the solution. The heat-generating body is covered with an air-permeable film or received in a flat-shaped bag. The material of the film or bag is chosen to be capable of supplying air necessary to the heat generation of the heat-generating body inside the cover or bag. A non-woven or woven fabric of a natural or synthetic fiber, a paper, a film of varying type of synthetic resin or a composite sheet may be used for the bag. Furthermore, the bag with the heat-generating body is stored in an outer sealed back of a non-air-permeable film until use.

US 5,187,814 A relates to a heated garment such as a mitten, glove or sock. The garment comprises an opening. An elongated chamber within the garment is accessible through the opening. A heater pack can be inserted through the opening into the elongated chamber. US 5,187,814 A does not disclose information concerning the design of the heater pack itself.

US 5,205,278 A discloses a chemical bag warmer being a sealed plastic bag containing a sodium acetate solution. A triggering member, here a disk-shaped element is disposed in the sodium acetate solution. The disk-shaped member generates an oscillation wave when a pressure is applied thereupon causing the triggering member to bend. The triggering member causes said sodium acetate solution to crystallize and to provide a warming effect through the dissipation of heat due to such crystallization.

US 5,084,986 A discloses a holder for a small-size disposable warmer of a chemical exothermic reaction type within the interior of footwear such as a shoe. US 5,084,986 A describes known disposable warmers basing upon an oxidation reaction of iron powder with the atmosphere. The holder for the disposable warmer comprises a permeable base member of a length which extends from a toe portion to a rear end portion at the mid-portion between the widest part and the heel part of the sole of a footwear. The permeabilities of the base member and of a closure member placed on the base member so as to wholly overlap the base member are different from each other. The disclosure of US 5,084,986 A focusses on the design of the holder. Concerning the disposable warmer, US 5,084,986 A discloses that the disposable warmer is contained in a bag made of cloth or fabric having an air permeability and contains an exothermic powder and a particle compound.

US 5,928,275 A relates to a body warming belt containing a number of pockets adapted to receive chemical or electrical heating devices at positions directly opposite the user's kidneys. Concerning the design of the heating device US 5,928,275 A refers to a heating pouch having a cover layer containing an oxygen-activated mixture which includes activated charcoal, iron powder, salt water and wood fibers with an operating life of about six hours. Furthermore, US 5,928,275 A discloses a battery powered heating unit.

### SUMMARY OF THE INVENTION

The invention is defined in the appended independent claim 1, preferred embodiments are described in the dependent claims.

Preferred embodiments of the present invention provide a self-contained disposable single-use heat generating apparatus. Briefly described, in architecture, one embodiment of the apparatus can be implemented as follows. A self-contained disposable single-use heat generating apparatus comprises a heat generating pack having a first bag layer bonded to a second bag layer creating a pouch therebetween. A heat generating agent is disposed in the pouch. At least a portion of one of the first bag layer and the second bag layer has an air permeable surface area with a predetermined airflow rate. The airflow rate through the air permeable surface area is predetermined such that the heat generating agent remains substantially evenly distributed within the pouch. Disclosed is also a method for providing therapeutic heat. In this regard, one embodiment of such a method, among others, can be broadly summarized by the following steps: containing a heat generating composition in a self-contained heat generating pack and introducing air to the heat generating composition such that the heat generating composition remains substantially evenly distributed within the heat generating pack.

Other systems, features, and advantages of the present invention will be or become apparent to one with skill in the art upon examination of the following drawings and detailed description. It is intended that all such additional systems, methods, features, and advantages be included within this description, be within the scope of the present invention, and be protected by the accompanying claims.

### BRIEF DESCRIPTION OF THE DRAWINGS

Many aspects of the invention can be better understood with reference to the following drawings. The components in the drawings are not necessarily to scale, emphasis instead being placed upon clearly illustrating the principles of the present invention. Moreover, in the drawings, like reference numerals designate corresponding parts throughout the several views.
FIG. 1 is a cutaway perspective view of an embodiment of the self-contained personal warming apparatus of the present invention.
FIG. 1A is a perspective view of an embodiment of a self-contained personal warming apparatus illustrated in FIG. 1.
FIG. 2 is a plan view of an embodiment of a bag layer of the apparatus illustrated in FIG. 1.
FIG. 3 is a plan view of an embodiment of a bag layer of the apparatus illustrated in FIG. 1.
FIG. 4 is a plan view of an embodiment of a bag layer of the apparatus illustrated in FIG. 1.

### DETAILED DESCRIPTION OF THE PREFERRED EMBODIMENTS

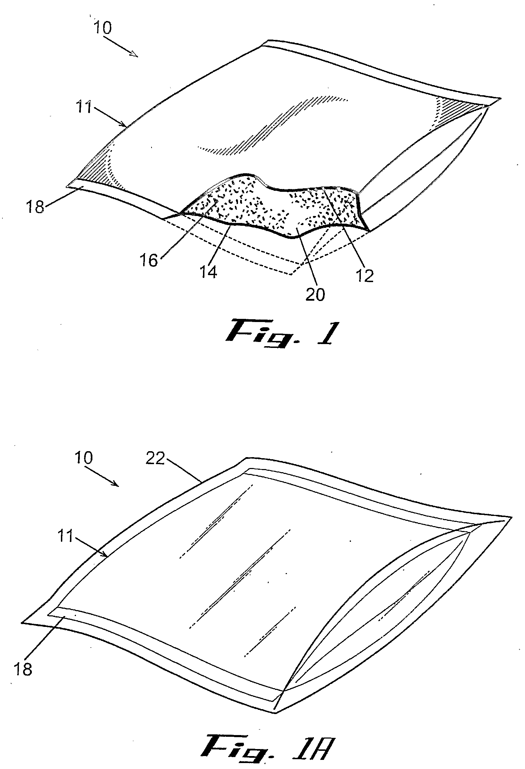

FIG. 1 illustrates one preferred embodiment of a self-contained disposable single-use heat generating apparatus 10 of the present invention. A heat generating pack 11 comprises a first bag layer 12, a second bag layer 14 and a heat generating agent 16 disposed therebetween. The first bag layer 12 is defined by a first set of dimensions and has a first surface area. The second bag layer 14 is defined by a second set of dimensions and has a second surface area. It should be noted that although the dimensions of the first bag layer 12 and the second bag layer 14 are illustrated as being substantially rectangular in shape, the dimensions can form any suitable shape. It is preferred, though not required, that the first surface area substantially corresponds to the second surface area.

The first bag layer 12 and the second bag layer 14 are aligned, one on top of the other, and are fixed together at a seam 18. The seam 18 can either extend around the perimeter of the heat generating pack 11 where the first bag layer 12 and second bag layer 14 meet, or run along one or a plurality of edges thereof. As illustrated in FIG. 1 the seam 18 runs along two opposing edges. The seam 18 is created in any suitable manner, for example by melting or bonding.

An enclosed space, or pouch 20, is created between the first bag layer 12 and the second bag layer 14. At least a portion of one of the first surface area and/or the second surface area are preferably air permeable as discussed in greater detail below. The first bag layer 12 and the second bag layer 14 preferably comprise a flexible fabric, material, or the like.

A heat generating agent 16 is disposed within the pouch 20 and contained therein. The heat generating agent 16 comprises a main ingredient of iron powder and incorporates therein, water, a water retaining material (charcoal, vermiculite, or the like), an oxidation promoter, such as activated carbon, and salt. More particularly, according to the invention, the agent 16 comprises approximately 35-50% by weight of iron powder, 25-45% by weight of water, approximately 10-14% by weight of water retaining agent, and approximately 4.5-6% by weight of salt. Upon exposure to air, oxidation of the iron begins in an exothermic reaction. The heat generated by the exothermic reaction of the agent 16 passes through the first bag layer 12 and the second bag layer 14 and radiates from the apparatus 10. The heat radiating from the apparatus 10 ranges from 39-45°C in order to provide a level of heat suitable for therapeutic heating without danger of burn to human skin.

During the exothermic reaction, the heat generating agent 16 consumes air at a predetermined air consumption rate. Controlling the rate of introduction of air to the heat generating agent 16 effects both the temperature radiated from the pack 11 as well as the shifting of the agent 16 within the pouch 20. Generally, the more air introduced to the heat generating agent 16, the hotter the pack 11 will become. Also, where the heat generating agent 16 consumes air faster than air is introduced to thereto, a vacuum will be created.

More specifically, and with reference to FIG. 1A, an embodiment of the self-contained disposable single-use heat generating apparatus 10 is illustrated. In this embodiment, the heat generating pack 11 is disposed inside a protective package 22. The protective package 22 can be hermetically sealed with the heat generating pack 11 inside such that no air or minimal air is introduced to the heat generating pack 11. In this embodiment, the protective package 22 effectively eliminates the introduction of air to the agent 16 thereby substantially preventing the heat generating exothermic reaction. The heat generating pack 11 is disposed within the protective package 22 preferably at, or closely after, the time of manufacture, and the heat generating apparatus 10 can be marketed, sold and stored in this configuration.

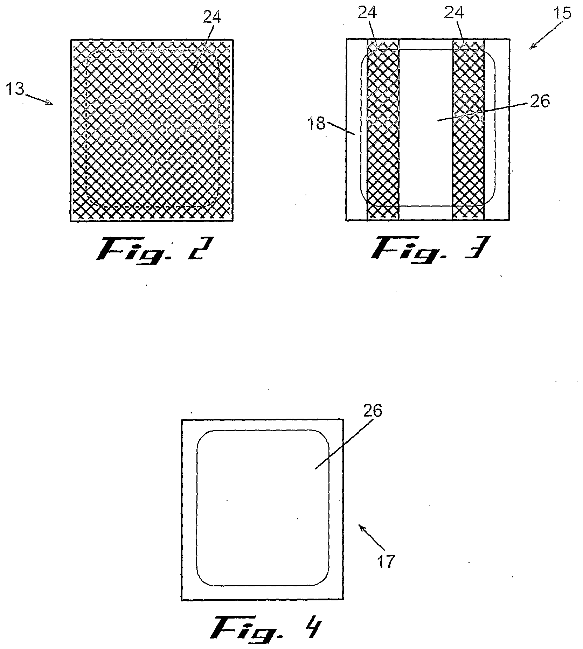

Referring next to FIGs. 2-4, various embodiments of bag layers 13, 15 and 17 are illustrated. The bag layers 13, 15 and 17 can comprise the first bag layer 12, the second bag layer 14 or any suitable combination thereof in order to form a heat generating pack 11. For example, a heat generating pack 11 can comprise a first bag layer 12 arranged in the configuration of bag layer 13 (FIG. 2) and a second bag layer 14 arranged in the configuration of bag layer 17 (FIG. 4).

Selection of the configuration of first bag layer 12 and second bag layer 14 is driven by a desired airflow rate for introduction of air to the heat generating agent 16. An air consumption rate of the heat generating agent 16 being at least slightly greater than an airflow introduction rate to the agent 16 generates at least a slight vacuum inside the pouch 20. The vacuum created inside the pouch 20 reduces shifting and settling of the heat generating agent 16, or "tea-bagging," within the pouch 20.

The substantially stationary disposition of the heat generating agent 16 inside the pouch 20 results in a heat generating pack 11 that maintains a substantially constant thickness. A substantially even heat profile is emitted across the surface area of the first bag layer 12 and the second bag layer 14. The airflow rate through the combined first surface area and second surface area of the first bag layer 12 and second bag layer 14 preferably is less than the predetermined air consumption rate of the heat generating agent 16 during exothermic reaction. For example, a heat generating pack 11 having porosity allowing an airflow rate of 20,000 sec./100 cc of air preferably contains a heat generating agent 16 having an air consumption rate greater than 20,000 sec./100cc of air during the exothermic reaction.

Referring more specifically to FIG. 2, one bag layer 13 configuration comprises an air permeable surface area 24. The air permeable surface area 24 preferably comprises a microporous fabric. A preferred microporous fabric can comprise a nonwoven fabric formed from individual fibers that are pressed together forming an interlocking web of fibers. The fibers can be fixed to each other either mechanically (for example, by tangling the fibers together) or chemically (for example, by gluing, bonding, or melting the fibers together). The present invention can comprise a microporous fabric known to one having ordinary skill in the art.

FIG. 3 illustrates a bag layer 15 configuration having a portion of the surface area thereof comprising an air permeable surface area 24 and a portion of the surface area comprising a air impermeable surface area 26. The air permeable surface area 24 preferably comprises a microporous fabric. A preferred microporous fabric for this configuration can comprise a nonwoven fabric formed from individual fibers that are pressed together forming an interlocking web of fibers. The fibers can be fixed to each other either mechanically (for example, by tangling the fibers together) or chemically (for example, by gluing, bonding, or melting the fibers together). This configuration can comprise a microporous fabric known to one having ordinary skill in the art. The air impermeable surface area 26 of the bag layer 15 can comprise polyethelene, polypropylene, or any suitable material. It is preferable that the air impermeable surface area 26 exhibits a low coefficient of friction, such as to allow the heat generating pack 11 to easily slide into a pocket (not shown) formed in a glove, sock, belt for holding heat generating packs in position, or the like. The preferred combination of air permeable surface area 24 and air impermeable surface area 26 of the bag layer 15 of FIG. 3 is determined by the desired air flow introduction rate to the heat generating agent 16 inside a pouch 11 this bag layer 15 configuration may be used to form.

FIG. 4 illustrates another bag layer 17 configuration. The bag layer 17 comprises an air impermeable surface area 26, such as polyethelene, or any suitable material. It is preferable that the air impermeable surface area 26 exhibits a low coefficient of friction, such as to allow the heat generating pack 11 to easily slide into a pocket (not shown) formed in a glove, sock, belt for holding heat generating packs in position, or the like.

Applying the above disclosed bag layer configurations 13, 15 and 17, heat generating packs 11 of various configurations can be formed. One configuration of a heat generating pack 11 comprises a first bag layer 12 comprising bag layer 13 configuration having an air permeable surface area 24 (illustrated in FIG. 2) and a second bag layer 14 comprising bag layer 17 having an air impermeable surface area 26 (illustrated in FIG. 4). In this configuration the rate at which air is introduced to the heat generating agent 16 is controlled by allowing a pre-determined flow rate through the first bag layer 12 and allowing substantially no air flow through the second bag layer 14.

Another configuration of a heat generating pack 11 comprises a first bag layer 12 comprising bag layer 13 having an air permeable surface area 24 (illustrated in FIG. 2) and a second bag layer 14 also comprising bag layer 13 also having an air permeable surface area 24 (illustrated in FIG. 2). In this configuration the rate at which air is introduced to the heat generating agent 16 is controlled by allowing a pre-determined flow rate through both the first bag layer 12 and the second bag layer 14.

A heat generating pack 11 of the present invention can also comprise a first bag layer 12 comprising bag layer 13 having an air permeable surface area 24 (illustrated in FIG. 2) and a second bag layer 14 comprising bag layer 15 having a portion of the surface area being air permeable 24 and a portion of the surface area being air impermeable 26 (illustrated in FIG. 3). In this configuration the rate at which air is introduced to the heat generating agent 16 is controlled by the total air permeable surface area 24 of the first bag layer 12 and the second bag layer 14 combined. It is preferable that the airflow rate through the total air permeable surface area 24 of the first bag layer 12 and the second bag layer 14 combined is less than the air consumption rate of the heat generating agent 16 during exothermic reaction.

A heat generating pack 11 of the present invention can also comprise a first bag layer 12 comprising bag layer 17 having an air impermeable surface area 26 (illustrated in FIG. 4) and a second bag layer 14 comprising bag layer 15 having a portion of the surface area being air permeable 24 and a portion of the surface area being air impermeable 26 (illustrated in FIG. 3). In this configuration the rate at which air is introduced to the heat generating agent 16 is controlled by the total air permeable surface area 24 of the second bag layer 14. It is preferable that the airflow rate through the total air permeable surface area 24 of the second bag layer 14 combined is less than the air consumption rate of the heat generating agent 16 during exothermic reaction.

It should be noted that the above described heat generating packs 11 are mere examples and that any configuration combining air permeable surface area 24 with air impermeable surface area 26 is within the spirit of the present invention.

In one method of use of an embodiment of a self-contained disposable single-use heat generating apparatus 10 of the present invention, a heat generating pack 11 is disposed in a protective package 22 to eliminate, or at least minimize, introduction of air to the heat generating agent 16 disposed inside the pack 11. The heat generating pack 11 is removed from the protective package 22. Air is introduced to a heat generating agent 16 disposed within a pouch 20 of the heat generating pack 11. The pouch 20 is formed by a first bag layer 12 and a second bag layer 14 being peripherally bonded to each other. The heat generating pack 11 is agitated, such as by shaking or crumpling the pack 11 in order to begin or speed up an exothermic reaction of the heat generating agent 16 with air. The heat generating agent 16 consumes air in a heat generating exothermic reaction, thereby emitting heat from the heat generating pack 11. At least one of the first bag layer 12 and the second bag layer 14, or a combination thereof, allow air to be introduced to the heat generating agent 16. The introduction of air is preferably at a flow rate less than the air consumption rate of the heat generating agent 16 during the exothermic reaction. The heat generating pack 11 can be positioned, as desired.

In one method of use, the heat generating pack 11 can be inserted into a pocket, for example a pocket disposed in a belt for heat application near a user's skin on their back, stomach, or any desired location. The heat generating pack 11 can also be inserted into a pocket formed in a sock or glove for a user to warm toes and fingers, respectively.

The exothermic reaction of the heat generating agent 16 when introduced to air produces a heat emission ranging between 39-45°C for approximately 12 to 18 hours. Upon the conclusion of the exothermic reaction and the cooling down of the heat generating pack 11, the heat generating pack 11 can be removed from the position at which it was placed for use and disposed.

It should be emphasized that the above-described embodiments of the present invention, particularly, any "preferred" embodiments, are merely possible examples of implementations, merely set forth for a clear understanding of the principles of the invention. Many variations and modifications may be made to the above-described embodiment(s) of the invention without departing substantially from the principles of the invention. All such modifications and variations are intended to be included herein within the scope of this disclosure and the present invention and protected by the following claims.

## Claims

1. A self-contained, disposable, single-use heat generating apparatus, comprising:
a heat generating pack comprising:
a first bag layer having a first surface area;
a second bag layer having a second surface area, said second bag layer being fixed to said first bag layer, such that said first bag layer and said second bag layer defining a pouch there between;
a heat generating agent disposed in said pouch, said heat generating agent arranged and configured to consume air at a predetermined air consumption rate in an exothermic reaction; and
at least one of said first surface area and said second surface area comprises an air permeable surface area having a predetermined airflow rate at which air is introduced to said heat generating agent, said predetermined airflow rate being arranged and configured to be less than said predetermined air consumption rate such that said heat generating agent remains substantially evenly distributed within said pouch, **characterized in that** the agent comprises
a) 35 - 50 % by weight of iron powder,
b) 25 - 45 % by weight of water,
c) 10 - 14 % by weight of water-retaining agent and
d) 4.5 - 6 % by weight of salt
and the heat radiating from the heat generating apparatus ranges from 39 - 45°C.

2. The apparatus of claim 1, wherein said first bag layer is defined by a set of dimensions substantially corresponding to a set of dimensions defining said second bag layer.

3. The apparatus of claim 1, wherein at least one of said first bag layer and said second bag layer comprises a microporous material.

4. The apparatus of claim 3, wherein said microporous material comprises a fabric having a plurality of fibers forming an inter-locking web, wherein at least a portion of said plurality of fibers are bonded to each other.

5. The apparatus of claim 1, wherein one of said first surface area and said second surface area comprises an air permeable surface area and the other of said first surface area and said second surface area comprises an air permeable surface area.

6. The apparatus of claim 1, wherein one of said first surface area and said second surface area comprises an air permeable surface area and the other of said first surface area and said second surface area comprises an air impermeable surface area.

7. The apparatus of claim 6, wherein said air impermeable surface area comprises a low coefficient of friction.

8. The apparatus of claim 1, further comprising:
a protective package for receiving said heat generating pack, said protective package being air impermeable and retarding said exothermic reaction.

9. The apparatus of claim 8, wherein said protective package is hermetically sealed with said heat generating pack disposed therein.

## Patentansprüche

1. In sich geschlossene, wegwerfbare Wärme erzeugende Vorrichtung für einen einmaligen Gebrauch, mit:
einem Päckchen zu Erzeugung von Wärme mit:
einer ersten Taschenlage mit einem ersten Oberflächenbereich;
einer zweiten Taschenlage mit einem zweiten Oberflächenbereich, wobei die zweite Taschenlage so an der ersten Taschenlage befestigt ist, dass die erste Taschenlage und die zweite Taschenlage zwischen sich einen Beutel definieren;
einem Mittel zur Erzeugung von Wärme, das in dem Beutel angeordnet ist, wobei das Mittel zur Erzeugung von Wärme so angeordnet und gestaltet ist, dass dieses bei einer exothermen Reaktion mit einer vorbestimmten Luftverbrauchsrate Luft verbraucht; und
wobei der erste Oberflächenbereich und/oder der zweite Oberflächenbereich einen luftdurchlässigen Oberflächenbereich mit einer vorbestimmten Luftflussrate aufweist, die so ausgelegt und gestaltet ist, dass diese kleiner ist als die vorbestimmte Luftverbrauchsrate, so dass das Mittel zur Erzeugung von Wärme in dem Beutel im Wesentlichen gleichmäßig verteilt bleibt,
**dadurch gekennzeichnet, dass** das Mittel
a) 35 - 50 % Gewichtsanteil Eisenpulver,
b) 25 - 45 % Gewichtsanteil Wasser,
c) 10 - 14 % Gewichtsanteil Wasserrückhaltemittel und
d) 4,5 - 6 % Gewichtsanteil Salz
aufweist und sich die Wärme, die von der Vorrichtung zur Erzeugung von Wärme ausgestrahlt wird, im Bereich von 39 - 45°C befindet.

2. Vorrichtung nach Anspruch 1, wobei die erste Taschenlage durch einen Satz von Abmessungen bestimmt ist, der im Wesentlichen einem Satz von Abmessungen entspricht, der die zweite Taschenlage bestimmt.

3. Vorrichtung nach Anspruch 1, wobei die erste Taschenlage und/oder die zweite Taschenlage ein mikroporöses Material aufweist.

4. Vorrichtung nach Anspruch 3, wobei das mikroporöse Material einen Stoff oder ein Gewebe aufweist, der oder das mehrere Fasern aufweist, die ein ineinandergreifendes Netz oder Gewebe bilden, wobei mindestens ein Teil der mehreren Fasern miteinander verbunden, verklebt oder stoffschlüssig verbunden sind.

5. Vorrichtung nach Anspruch 1, wobei der erste Oberflächenbereich oder der zweite Oberflächenbereich einen luftdurchlässigen Oberflächenbereich aufweist und der andere von dem ersten Oberflächenbereich und dem zweiten Oberflächenbereich einen luftdurchlässigen Oberflächenbereich aufweist.

6. Vorrichtung nach Anspruch 1, wobei der erste Oberflächenbereich oder der zweite Oberflächenbereich einen luftdurchlässigen Oberflächenbereich aufweist und der andere von dem ersten Oberflächenbereich und dem zweiten Oberflächenbereich einen luftundurchlässigen Oberflächenbereich aufweist.

7. Vorrichtung nach Anspruch 6, wobei der luftundurchlässige Oberflächenbereich einen niedrigen Reibungskoeffizienten aufweist.

8. Vorrichtung nach Anspruch 1, die weiterhin aufweist:
eine Schutz-Verpackung zur Aufnahme des Päckchens zur Erzeugung von Wärme, wobei die Schutz-Verpackung luftundurchlässig ist und die exotherme Reaktion verzögert.

9. Vorrichtung nach Anspruch 8, wobei die Schutz-Umhüllung hermetisch abgeschlossen ist, wobei sich das Päckchen zur Erzeugung von Wärme darin befindet.

## Revendications

1. Appareil générateur de chaleur autonome, jetable et à usage unique, comprenant :
une garniture génératrice de chaleur comprenant :
une première couche formant sac ayant une première surface ;
une seconde couche formant sac ayant une seconde surface, ladite seconde couche formant sac étant fixée à ladite première couche formant sac, de sorte que ladite première couche formant sac et ladite seconde couche formant sac définissent une poche entre elles ;
un agent générateur de chaleur disposé dans ladite poche, ledit agent générateur de chaleur étant agencé et conçu pour consommer de l'air à un taux de consommation d'air prédéterminé dans une réaction exothermique ; et
au moins une de ladite première surface et de ladite seconde surface comprend une surface perméable à l'air ayant un débit d'air prédéterminé auquel l'air est introduit dans ledit agent générateur de chaleur, ledit débit d'air prédéterminé étant agencé et conçu pour être inférieur audit taux de consommation d'air de sorte que ledit agent générateur de chaleur reste réparti de manière sensiblement uniforme à l'intérieur de ladite poche,
**caractérisé en ce que** l'agent comprend
a) de 35 à 50 % en poids de poudre de fer,
b) de 25 à 45 % en poids d'eau,
c) de 10 à 14 % en poids d'un agent de rétention d'eau et
d) de 4,5 à 6 % en poids de sel
et **en ce que** la chaleur émanant de l'appareil générateur de chaleur varie de 39 à 45°C.

2. Appareil selon la revendication 1, dans lequel ladite première couche formant sac est définie par un ensemble de dimensions correspondant sensiblement à un ensemble de dimensions définissant ladite seconde couche formant sac.

3. Appareil selon la revendication 1, dans lequel au moins une de ladite première couche formant sac et de ladite seconde couche formant sac comprend un matériau microporeux.

4. Appareil selon la revendication 3, dans lequel ledit matériau microporeux comprend un tissu ayant une pluralité de fibres formant une bande à emboîtement, dans lequel au moins une partie de ladite pluralité de fibres sont liées les unes aux autres.

5. Appareil selon la revendication 1, dans lequel une de ladite première surface et de ladite seconde surface comprend une surface perméable à l'air et l'autre de ladite première surface et de ladite seconde surface comprend une surface perméable à l'air.

6. Appareil selon la revendication 1, dans lequel une de ladite première surface et de ladite seconde surface comprend une surface perméable à l'air et l'autre de ladite première surface et de ladite seconde surface comprend une surface imperméable à l'air.

7. Appareil selon la revendication 6, dans lequel ladite surface imperméable à l'air comprend un faible coefficient de frottement.

8. Appareil selon la revendication 1, comprenant en outre :
un emballage de protection pour recevoir ladite garniture génératrice de chaleur, ledit emballage de protection étant imperméable à l'air et retardant ladite réaction exothermique.

9. Appareil selon la revendication 8, dans lequel ledit emballage de protection est hermétiquement scellé avec ladite garniture génératrice de chaleur disposée à l'intérieur.
